# EUROPEAN PATENT APPLICATION

(11) **EP 1 777 520 A1**
(43) Date of publication of application: **25.04.2007**
(21) Application number: 06450145.5
(22) Date of filing: 16.10.2006
(51) Int. Cl.: G01N 33/543

(54) **Methods for manufacturing supported lipid membranes**

(30) Priority: 19.10.2005 AT 17112005
(71) Applicant: Universität für Bodenkultur Wien, 1180 Wien (AT)
(72) Inventor: Sleytr, Uwe B., 1220 Vienna (AT); Pum, Dietmar, 1060 Vienna (AT); Schuster, Bernhard, 1170 Vienna (AT); Gufler, Petra, 1190 Vienna (AT)
(74) Representative: Sonn & Partner Patentanwälte

(57) **Abstract**

Method for the manufacturing of a supported lipid membrane comprising the steps:
- providing a substrate, covered at least in part with a layer comprising proteins, glycoproteins, polypeptides and/or peptides,
- contacting and incubating said substrate with a solution comprising bicelles, which comprise at least one short chain and at least one long chain phospholipid and/or at least one detergent, thereby producing a supported lipid membrane, and
- optionally removing the lipid membrane formed on the substrate from said substrate.

## Description

The present invention relates to methods for manufacturing supported lipid membranes.

Membrane proteins have been identified as key targets for biomedical research. In order to understand numerous cellular processes mediated by membrane proteins, information about their structure, function and their involvement in various diseases is essential. Combinatorial genetics and chemistry provide a large number of pharmaceutical molecules and genetically engineered proteins to be tested. Thus, there is great demand for automated, efficient and reliable high-throughput screening tools - especially on single ion channel- and receptor activity - not only for drug discovery efforts, safety screening and quality assurance, but also in basic research (proteomics).

In order to study the functioning of membrane associated proteins, which are anchored to lipid membranes or span through said membranes, under reproducible conditions a lipid bilayer simulating naturally occurring biological membranes has to be provided. Since patch-clamp and free-standing lipid membrane techniques cause stability problems, different types of stabilized or supported lipid membranes and methods obtaining such membranes were developed (Sackmann, 1996; Cornell et al., 1997; Schiller et al., 2003). One of the most wide spread techniques in the art for producing lipid membranes is the Langmuir Blodgett (LB) technique (see e.g. Zasadzinski JA, et al. Science (1994) 263:1726-1733). With said method it is possible to obtain Langmuir Blodgett films (mainly lipid monolayers) as a mechanically assembled array of amphiphillic molecules upon a water surface. Once the molecules are compressed to the desired organization, the film can then be transfered to a solid support, which may also comprise openings, by removing said solid support previously brought in the dipping well of a Langmuir-Blodgett apparatus from said well. Based on the dipping technique solid supported lipid films with different physical properties may be obtained. An alternative approach to manufacture solid supported lipid membranes is the use of liposomes. Liposomes obtained by commonly known methods may be fused on the surface of a solid support (Keller et al., 1998). The formation of planar lipid membranes on a solid support with liposomes is a two-step process. First, the liposomes have to adsorb on the substrate and in a second step bilayer formation has to occur by fusion of the liposomes. The above described method, however, frequently suffers from the lack of reproducible bilayer formation as adsorbed liposomes remain intact and cover the surface of the substrate. This lipid structure is not suited for electrophysiological investigations on single membrane proteins.

The WO 01/81425 discloses the use of secondary cell wall polymer for immobilising S-layer proteins on a surface of a substrate. The S-layer coated surfaces obtained by such a method can be used to further produce lipid films.

Pum D et al. (Trends in Biotechnology 17 (1999):8-12) disclose potential applications of S-layer proteins.

The WO 02/095406 relates to methods for producing immobilised lipid double layers on the surface of a substrate.

In the US 4,921,706 unilamellar lipid vesicles comprising short-chain and long-chain phosphorlipids are described.

Since all these methods for manufacturing solid supported lipid membranes are labour intensive and do not result in lipid membranes with a satisfactory quality required to perform studies on e.g. membrane related proteins it is an object of the present invention to provide a new method for manufacturing lipid membranes, in particular solid supported lipid membranes, which may overcome the disadvantages of the methods of the state of the art.

Therefore, the present invention provides a method for the manufacturing of a supported lipid membrane comprising the steps:
- providing a substrate, covered at least in part with a layer comprising proteins, glycoproteins, polypeptides and/or peptides,
- contacting and incubating said substrate with a solution comprising bicelles, which comprise at least one short chain and at least one long chain phospholipid and/or at least one detergent, thereby producing a supported lipid membrane, and
- optionally removing the lipid membrane formed on the substrate from said substrate.

In order to manufacture lipid membranes with or without proteins (e.g. enzymes, ion channels) being anchored to or spanning said membranes an appropriate substrate has to be provided, which allows the formation of a substantially regular lipid membrane. It surprisingly turned out that not only the selection of an appropriate substrate is important but that especially the choice of a suitable lipid membrane structures to be fused on a solid support in order to get a lipid membrane is of major importance. Suitable lipid membranes structures according to the present invention are bicelles. In Erb E.-M. et al. (Anal Biochem 280:29-35 (2000)), for instance, the use of bicelles and liposomes for the manufacture of lipid membranes on a substrate comprising a dextran matrix with hydrophobic residues on its surface is described. After incubation with liposomes or bicelles, a significant decrease in binding of BSA on the hydrophobic dextran matrix has been observed. The authors suggest that liposomes as well as bicelles are both equally well suited for obtaining lipid membranes on such substrates. However, it is well known in the art that on substrates covered with proteins, e.g. S-layer proteins (Wetzer, 1997), glycoproteins, polypeptides and/or peptides (Naumann et al., 1999, 2002) no lipid membranes with sufficient electrochemical properties, stability and quality can be obtained by using liposomes. Therefore, it was surprising that bicelles can be used to manufacture lipid membranes with sufficient electrochemical properties, stability and quality on such substrates.

"Bicelles" according to the present invention are disc-shaped lipid aggregates composed of a binary mixture of long-chain phospholipids and at least one short chain phospholipid and/or at least one detergent. (Whiles et al., 2002; Raffard et al., 2000; Glover et al., 2001). The planar region is composed of long-chain phospolipids, which can be doped with phospholipids with different headgroups to alter the charge characteristics of the membrane (Struppe et al., 2000). The bicelle rim is stabilized by a surfactant. Originally, bicelles were introduced by Prestegard and co-workers as a membrane model for solid-state NMR studies of membrane-associated biomolecules (Ram and Prestegard, 1988; Sanders and Prestegard, 1990). More recently, bicelles were used to study the functionality of complex membrane proteins reconstituted in bicelles (Sanders and Landis, 1995; Sasaki et al., 2003) and in protein crystallization (Faham and Bowie, 2002; Faham et al., 2005).

"Detergents" according to the present invention are amphipathic, surface active, molecules with polar (water soluble) and nonpolar (hydrophobic) domains. They bind strongly to hydrophobic molecules or molecular domains to confer water solubility.

"Lipid membrane" according to the present invention is intended to be a lipidic structure characterized by a hydrophobic core (alkyl chains) and two hydrophilic outer parts (head group regions). Lipid membranes are intended to be built by a lipid bilayer composed of phospholipids and/or etherlipids or by a tetraetherlipid monolayer. However, according to the present invention also multiple piled lipid layers, one upon the other, are lipid membranes as defined herein.

In the context of the present invention the term "substrate" is referred to any material suited to support lipid membranes and resulting consequently in "supported lipid membranes". "Substrate" is not only restricted to solid supports but includes also material which may comprise lipid membranes or protein layers.

Optionally the lipid membrane formed on the substrate may be transferred to another solid support or may be used at least partially without any solid support (e.g. as a barrier between two compartments of a container connected via an opening) (Gufler et al. 2004, Schuster et al. 2001, Schuster et al., 2003).

The binding of bicelles to a substrate is influenced by the chemical and physical properties of said substrate. Due to the presence of charged phospholipids in bicelles, bicelles tend to bind electrostatically to substrates exhibiting a high charge density. The fusion of single bicelles on a substrate to a lipid membrane is influenced by the presence of hydrophobic regions on said substrate. These hydrophobic regions allow the formation of lipid monolayers from bicelles on the substrate which consequently catalyse the growth of the lipid membrane on the substrate. Since proteins, glycoproteins, polypeptides and peptides exhibit these characteristic features and are able to catalyse hydrophobic as well as electrostatic interactions with other molecules the substrate is covered at least in part with said molecules.

The immobilisation of proteins, glycoproteins, polypeptides and/or peptides on substrates provide a certain kind of spacer between the substrate and the lipid membrane. The provision of a gap between the substrate and the lipid membrane is important for the realisation of experiments, like electrophysiological measurements. In the art several methods are disclosed which allow the formation of said gap. These methods include e.g. the provision of a hydrogel (e.g. dextran) on a solid support, wherein on said hydrogel the lipid membrane is brought up (Erb E.-M. et al. (2000) Anal Biochem 280:29-35). The most sophisticated concept is the application of bifunctional molecules instead of a polymer cushion, providing a lipophilic domain and a hydrophilic spacer. The lipophilic part inserts into one or both leaflets of the lipid membrane and can consist of phospholipids (Naumann et al., 2002; Peggion et al., 2001), cholesterols (Lang et al., 1994), alkyl chains (Cornell et al., 1997), or phytanoyl groups (Schiller et al., 2003). The hydrophilic spacer anchors the tethering molecule to the support and determines the hydrophilic environment as well as the volume of the sub-membraneous space. Most of the tether molecules are linked by the thiol chemistry on gold surfaces or are coupled by cross-linker to oxide surfaces. A broad spectrum of molecules like lipids (thio-, histidine-, and succinimidyl-lipids), peptides, oligomers, polymers, or carbohydrates are used to generate the tethering layer. However, the tethering part requires some spacer molecules to control the lateral spacing between the tether molecules to obtain a functional, well-defined ionic reservoir on which the membrane rests (Raguse et al, 1998). Tethered bilayer lipid membranes (tBLMs) address the necessity of a sub-membraneous space serving both as an ionic reservoir as well as providing adequate space for incorporated membrane proteins (Krishna et al., 2003). The most demanding problem in tBLMs is to achieve electrical properties which are competitive with free-standing lipid membranes. Moreover, it has to be mentioned that in particular the less leaky systems often can not be functionalized by membrane proteins, most probably due to the limited fluidity of these tBLMs.

According to the present invention "layer comprising proteins, glycoproteins, polypeptides and/or peptides" includes all kind of proteins, glycoproteins, polypeptides and/or peptides which may be immobilised on the surface of a substrate. Furthermore, this definition refers not only to mono-layers but includes also protein multi-layers. Protein multi-layers represent piled mono-layers (e.g. two, three, five or more protein monolayers form a protein multilayer). Furthermore, the protein, glycoprotein, polypeptide and/or peptide may be homogeneous and consequently comprise molecules of the same type or heterologous and comprise mixtures of proteins, glycoproteins, polypeptides and/or peptides of different species.

According to a preferred embodiment of the present invention the protein, glycoprotein, polypeptide and/or peptide layer is modified with at least one functional group, wherein the at least one functional group is preferably selected from the group consisting of proteins, polypeptides, peptides, glycans and mixtures thereof.

The use of a modified and/or recombinant protein layer on the substrate will provide functionalities to which bicelles could be bound either via chemical coupling reactions (e.g. ester-linkage) or specific interactions (e.g. S-layer/streptavidin fusion protein layers in combination with biotinylated bicelles). Other possible specific interactions are lectine linkages (dextrane-ConA, asialoglycoprotein receptor-glucose), affinity linkages (e.g. his-tag/Ni2+-ions/chelator), ligands-receptor interactions, S-layer-SCWP-interactions (AU 5,201,001). Of course, it is also possible to employ proteins and polypeptides with amino acid modifications as a result of recombinant amino acid exchange (e.g. basic to acidic amino acids) or chemical modifications (e.g. cross-linking, deviation). Such proteins may exhibit different physico-chemical properties (e.g. surface charge) than the corresponding protein and may be used to bind bicelles to the substrate and "catalyse" the formation of lipid membranes more or less efficiently.

The protein/glycoprotein layer is preferably composed of S-layer proteins or fragments thereof, which can still form a regular protein layer comparable to native S-layer proteins. The S-layer protein lattice with its repetitive physicochemical properties down to the sub-nanometer range provides anchoring sites for the lipid membranes (stabilizing effect) but retaining a lateral diffusion of the lipid molecules (fluidity) that does not impair the incorporation of even large membrane proteins. In addition, membrane proteins can also be anchored on the S-layer lattice, particularly on S-layer fusion proteins (e.g. S-layer/streptavidin fusion proteins interact with a biotinylated lipid membrane protein). This will allow a controlled orientation of the membrane protein in the lipid membrane.

S-layer proteins as substrates or covering a substrate are very well suited for the manufacture of lipid membranes, because their ability to form regular shaped structures allows to provide appropriate surfaces on which lipid membranes can be formed with bicelles. In particular, the smoothness of the S-layer lattice with steps not higher than the thickness of one protein monolayer (e.g. ~ 5 nm to 8 nm) renders the fabrication of highly flat lipid membranes. In addition, the crystalline S-layer provides repetitive functional groups in well-defined positions. Thus, the S-layer enables a well-defined and high-oriented binding of membrane-related molecules (membrane proteins, lipids. In contrast, even in the case of ultrathin polymer cushions, steps of up to 100 nm and more can be expected. Dextran matrices are highly rough and amorphous structures and chemically introduced functional groups like hydrophobic residues are allocated randomly. It is concluded, that the smoothness of the S-layer lattices exerts a strong positive effect on the stability and fluidity of supported lipid membranes. Most important, the fusion is thought to be facilitated due to a better accessibility of the bicelles to each other.

"S-layer" proteins are the outermost cell envelope component of a broad spectrum of bacteria and archaea. S-layers are composed of a single protein or glycoprotein species (Mw 40-200 kDa) and exhibit either oblique (p1, p2), square (p4) or hexagonal (p3, p6) lattice symmetry with unit cell dimensions in the range of 3 to 30 nm. S-layers are generally 5 to 10 nm thick and show pores of identical size (diameter, 2 - 8 nm) and morphology. S-layer proteins are routinely utilised in several technical applications. For instance the EP 0 306 473 B1 discloses the use of S-layer proteins as a carrier for haptens, immunogenic or immunostimulant substances. The immobilisation of molecules, particularly of proteins, on S-layer proteins is described in the EP 0 362 339 B1. In the WO 02/097118 A1 the production of a layer of functional molecules on a substrate using S-layer proteins is disclosed. Therein the protein layer on the surface of a carrier is formed by creating an electrochemical potential difference between the solution and the surface. The EP 0 189 019 B1 discloses the making and the use of an ultrafiltration membrane employing S-layer proteins.

Also the application of two-dimensional crystalline bacterial surface layers (S-layers) as molecular building blocks within the scope of solid supported functional phospholipid and tetraetherlipid membranes has been introduced (Schuster et al., 1998, 2001, 2003; Gufler et al., 2004). The state of the art recommends S-layers as promising structures for new "smart" membrane-based biosensors with an enhanced mechanical long-term robustness, ease of formation and low fabrication costs.

In particular substrates covered at least in part with S-layer proteins turned out to be especially suitable for the manufacturing of lipid membranes. Substrates covered with S-layer proteins show gaps, steps, defects and crystallite boundaries, where two sheets of the upper S-layer lattice meet. Such irregularities in the topography of the substrate surface act as lipid binding sites and are recognized by bicelles. In a first self-assembly process (SA1) the bicelles adhere to the lipid binding sites. In a second self-assembly process (SA2) bound bicelles act as starting points for subsequent lateral bilayer growth by fusion of bicelles to the preexisting bilayer. SA1 is assumed to be a bicelle-S-layer interaction, whereas SA2 mainly is a predominantly bicelle-bicelle-interaction. While interactions between lipid aggregates (e.g. liposomes, bicelles, bilayers) are well characterized (Stryer, 1995), lipid aggregate-S-layer interactions are still poorly understood. Little is known about the 3-dimensional molecular configuration of S-layer proteins and the arrangement of their functional groups. On both, mono- and bilayered S-layer proteins (e.g. SbpA: NCBI Acc. No. AAF22978), the outer protein surface is exposed to the aqueous bulk solution. Bicelle binding and bilayer growth occurs not only with mono-layered S-layer lattices but also with bi- or multi-layered S-layer lattices (e.g. bilayered S-layer lattices). The lipid binding sites are formed by functional groups which are located either on the top or on the bottom lattice.

There are several possible mechanisms for bicelle adhesions to "S-layer lipid binding sites".
(i) A common feature of S-layers of many Bacillaceae is their smooth, charge neutral outer surface and a more corrugated inner surface of high charge density (Pum and Sleytr, 1996). The inner surface of several S-layer proteins is net-positively charged (Gyärvary et al., 2003). Phosphatidylcholine headgroups bind to surfaces with high charge density. Crosslinking the proteins aminogroups with the strong crosslinker glutaraldehyde resulted in a further decrease in bicelle binding. Incubation of the crystalline S-layer protein with the homobifunctional cross-linker BS³ (bis (sulfo-succinimidyl) suberate) contributed also in bilayer formation, especially for longer incubation times. BS³ consists of two reactive sulfo NHS ester groups which are reactive towards amino groups on the surface of proteins. The spacer arm separating the two ester groups is 1.14 nm in length. Consequently, electrostatic interactions contribute to bicelle adhesion.
(ii) Alternatively protein-epitopes may exist to which the lipid headgroups fit. Such epitopes can be formed by several amino acid residues. The crystalline properties of S-layers and the alignment of functional groups in well defined positions would preserve a dense and repetitive arrangement of such epitopes.
(iii) Remnants of secondary cell wall polymer (SCWP) on native S-layer proteins may also initiate bicelle adhesion.
(iv) Hydrophobic interactions may play a decisive role in the folding and assembly of proteins as well as in lipid bilayer formation (Stryer, 1995). Isolated S-layer proteins reassemble spontaneously into a crystalline two-dimensional array on a great variety of surfaces and interfaces. The driving forces in this self-assembly process are non-covalent interactions, predominantly hydrophobic interactions. Hydrophobic domains along the defect rims, steps and crystallite boundaries of recrystallized multilayered S-layer lattices might be accessible and interact with the hydrophobic bicelle core. Due to the repetitive properties of S-layers the hydrophobic domains form a "repetitive hydrophobic pearl necklet" along the defect zones. When a bicelle approaches, it might form a partial lipid monolayer on the hydrophobic areas of the S-layer, whereas on the hydrophilic areas the bicelle remains bilayered.
(v) From a geometrical point of view - structural and edge- related effects that are ascribed to lattice defects and irregularities, gaps, steps and crystallite boundaries in an S-layer lattice may also contribute to bicelle adhesion. Lattice irregularities as well as the crystallite size of an S-layer are influenced by the used S-layer proteins and the surface properties of the substrate. The edge geometry and the level determine the dimension, the roughness and the sharpness of the gaps and steps as well as the density and regularity of the lipid binding sites.

The mechanisms of bicelle binding are mainly an interplay of electrostatic and hydrophobic interactions which are determined and quantified by the intrinsic properties of the protein, glycoprotein, polypeptide and peptide layer, in particular of the S-layer.

Although both bicelles and liposomes seem to recognize lipid binding sites on a multilayered protein, glycoprotein, polypeptide and peptide lattice, the use of liposomes in order to obtain (solid supported) lipid membranes is not as suited as the use of bicelles. Liposome fusion is a frequently used method to prepare membranes on solid supports. Vesicles in solution show a tendency to deposit on surfaces (Seifert and Lipowsky, 1991). When liposomes encounter a solid surface, they may adsorb and either remain intact (adsorption) or rupture and spread to form a lipid bilayer (fusion) (Jass et al., 2000). In comparison to bicelles, liposomes only adsorb and remain intact.

The quantities of various parameters that contribute to vesicle-protein interactions and vesicle fusion are still unknown. Vesicle rupture and fusion on solid supports is very complex and strongly depends on the surface properties, the surface-liposome interaction, the composition, the size, the charge, solution properties of the liposomes, the temperature and osmotic stress (Jass et al., 2000; Reimhult et al., 2003; Seitz et al., 2000). It turned out that the bicelle morphology itself is an important factor for the successful formation of solid supported lipid membranes. Combining bicelles with a substrate covered at least in part with proteins, glycoproteins, polypeptides and peptides, in particular with S-layer proteins, is an entirely new method. No other lipid aggregate (e.g. liposomes, micelles) delivered the same result to date. The lipid-membranes obtained by a method according to the present invention are stable for more than two, preferably for more than three, in particular for more than four weeks, at 4°C. The implementation of bicelle fusion appears to be a simple and practicable method for producing supported membranes, which may be used for the manufacturing of protein, glycoprotein, polypeptide and peptide as well as S-layer-covered microarrays with respect to membrane/protein-based biosensors for high-throughput-screening and lab-on-a-chip-diagnostics. Rapid and reliable screening of membrane protein functions provide biologically relevant information of utmost importance for people working in the fields of e.g. pharmaceutical research, quality control and proteomics. With regard to single ion channel measurements, highly insulating membranes are required.

Protein, glycoprotein, polypeptide and peptide supported membrane chips are applicable to a great variety of techniques such as electrochemical techniques (e.g. impedance spectroscopy), piezo-electric crystals and surface acoustic devices (e.g. QCM-D), surface plasmon and waveguide devices (e.g. SPR, SPR-imaging), fluorescence techniques (e.g. TIRF, FRET, and fluorescence microscopy), immunoanalytical techniques and techniques using thermistors (e.g. detection of exothermic membrane-related reactions). The broad spectrum of methods allows diversified fields of application such as screening of pharmaceutical molecules, investigation of structure-function- relationship of membrane proteins, ligand-receptor interactions (e.g. ligand fishing, investigation of hormonal signal transduction, affinity constants, binding specificities), monitoring of the active and passive transport of ions and molecules across the membrane and screening of toxic compounds in quality control and safety screening.

According to the present invention S-layer protein species from different organisms can be used. A protein species is used as a native, a recombinant, or a recombinant protein optionally with distinct functional domain(s), e.g. streptavidin, ligand, affinity tag. The S-layer is composed of identical S-layer proteins, a mixture of at least two species, or different protein species with each comprising one or more specific functionalities. The ratio of the distinct proteins can be varied. The S-layer can be native or chemically modified. Furthermore S-layers can be recrystallized as mono-, bi- or multilayers.

Dependent on the S-layer protein species employed, distinct surface properties are obtained after recrystallization:
*) intrinsic functionalities of native isolated S-layer proteins and recombinant S-layer proteins with no fused functional domain: e.g. charge density and distribution. In many cases the recombinant S-layer proteins that are predominantly expressed in E. coli show a different charge distribution than the native protein isolated from the bacterial cell wall. With regard to their orientation in vivo, a common feature of S-layers is their smoother charge-neutral outer surface and a more corrugated, net-negatively charged inner surface. Hence, the orientation of the S-layer proteins on the substrate defines the physico-chemical properties.
*) functionalities after chemical surface modification of native S-layers; e.g. change in charge and charge density, introduction of chemically reactive groups.
*) artificially inserted functional domains : genetically engineered S-layer fusion proteins, chemically fused functional domains.
*) inert surface character: neutrally charged surfaces. These types of S-layers show no binding affinity or unspecific binding.

Functional groups of the at least one functionality on S-layers interact with the lipid head group region of phospholipids, leading to a stabilization of lipid membranes. Stabilization was demonstrated on lipid membranes (Wetzer et al., 1997, 1998; Gufler et al., 2004) and on liposomes (Küpcü et al., 1995; Mader et al., 1999). S-layers do not penetrate the hydrophobic region of mono- (e.g. tetraetherlipid) or bilayer membranes; therefore they have no impact on the integrity of the lipid aggregates, cells or cell membranes. S-layers provide a natural environment for lipid membranes and biological materials: e.g. in many archaeal organisms the S-layer is the only component external to the cytoplasmic membrane. A broad range of S-layer proteins with different physico-chemical surface properties and functionalities are available. The implementation of recombinant S-layer- (US 2002/0168728 A1) and S-layer-fusion proteins exhibiting functional domains, enables the application of specific binding mechanisms (Moll et al., 2002). Due to the high regularity of the crystal lattice, functionalities repetitively arranged in well defined positions and orientations can be obtained. Furthermore a significant reduction in surface roughness of the substrate is observable after recrystallization (Gufler et al., 2004, Schuster et al., 2003).

The substrate is preferably substantially planar.

Although a substantially planar form of the substrate is preferred, it is of course also possible to use substrates which may have any three dimensional structure. Such substrates include also substrates with e.g. a spherical shape.

According to a preferred embodiment of the present invention the substrate comprises a material selected from the group consisting of silicon, semiconducting material, in particular semiconducting silicon, gallium arsenide and aluminum gallium arsenide, organic or inorganic polymers, solid state polymers and mixtures thereof.

The surface of the substrate is preferably coated with a metal and/or metal oxide.

It is advantageous that the substrate on which the protein layer is immobilized is covered by a metal and/or metal oxide. The metallisation of the substrate may be done by sputtering metal on the surface or by electrochemical methods. Furthermore, a homogeneous metallisation of the surface will ensure that physical properties such as conductivity of the surface will be substantially identical throughout the surface of the substrate.

The metal which is applied to the substrate surface is preferably gold or aluminium and the metal oxide is preferably indium tin oxide (ITO).

It is known in the art that due to its physico-chemical properties especially gold allow the immobilisation of proteins (e.g. S-layer proteins) and other chemical groups on surfaces. Especially surfaces coated with gold are preferred, because such substrates with lipid layers bound thereto may be used in various surface sensitive techniques, like surface plasmon resonance spectroscopy (SPR). To improve the adhesion of gold on the surface of a substrate, said surface may additionally be coated with chromium before gold is applied.

According to a preferred embodiment of the present invention the surface of the substrate is chemically or physically modified.

Due to the introduction of modifications on the surface of the substrate it is possible to change the chemical and/or physical properties of said surface. Furthermore, it is also possible to functionalise the surface in order to get e.g. a surface with distinct functional groups. Consequently, the surface may become more hydrophobic or more hydrophilic compared to an untreated surface or may be able to bind specifically molecules to the surface (e.g. antibodies).

The modification is preferably selected from the group consisting of ionizing radiation, atomic radicals, corona treatment, silane groups, functional groups and mixtures thereof.

The functional group is preferably a glycan chain, in particular a secondary cell wall polymer.

Glycan chains may be used to immobilise the protein layer or parts of said layer on a surface provided that the proteins are able to bind to said glycan chains (e.g. lectines). S-layer proteins, for instance, are able to bind to glycan chains like secondary cell wall polymers. In the WO 01/81425 A1 the use of a structure comprising carbohydrates (secondary cell wall polymer) as an anchoring means for S-layer proteins in order to bind them to a solid support is disclosed. Therefore, it is advantageous if the substrate is intended to be covered e.g. with s-layer proteins to functionalise the surface of the substrate with secondary cell wall polymers.

According to a preferred embodiment of the present invention the at least one long chain phospholipid comprises fatty acid residues with 10 to 24, preferably 12 to 20, more preferably 14 to 18, carbon atoms, wherein the at least one long chain phospholipid is preferably selected from the group consisting of dimyristoylphosphatidylcholine (DMPC), dimyristelaidoylphosphatidylcholine, myristoylpalmitoylphosphatidylcholine, dipalmitoylphosphatidylcholine (DPPC), 1,2-bis(10,12-tricosadiynoyl)-sn-glycero-3-phosphocholine, di-O-tetradecylphosphatidylcholine, egg-PC, di-O-hexadecylphosphatidylcholine and mixtures thereof. The long chain phospholipid may be mixed with or substituted by tetraetherlipids preferably selected from the group consisting of caditocalarchaeol tetraetherlipids, in particular glycerol-dialkyl-nonitol-tetreather lipid (GDNT) and glycerol-dialkyl-glycerol-tetreather lipid (GDGT), calarchaeol tetraetherlipids and mixtures thereof. Tetraetherlipids may preferably employed to create stable lipid membranes.

Tetraetherlipids are located in the cell membrane of the archaeal bacteria like the archaeon Thermoplasma Acidophilum. These archaebacterial lipids can be isolated in relatively high amounts and purified by chromatography. The headgroups can be modified selectively to obtain a covalent fixation to the substrate as well as to establish different surface properties regarding to the physico-chemical character (hydrophobicity, surface charge, surface energy), the biomimetic character (e.g. Phosphorylcholine) and/or to influence the specific surface composition (e.g. coupling of specific signal molecules, peptides, etc.). Surfaces coated with tetraetherlipids are very stable against oxydative, enzymatic and hydrolytic degradation. Furthermore, steam as well as gamma sterilisation are possible. Lipid membranes comprising tetratetherlipids can be applied for anti-stick coatings, biofilm protection or antifouling surfaces, biocompatible surfaces, specific sensor or biological sensor coatings as well as molecular films (e.g. for electrophysiological studies).

According to another preferred embodiment of the present invention the at least one short chain phospholipid comprises fatty acid residues with 4 to 10, preferably 6 to 8, carbon atoms, wherein the at least one short chain phospholipid is preferably selected from the group consisting of dicaproylphosphatidylcholine (DHPC), dicapryloylphosphatidylcholine (DCPC), dicaprylphosphatidylcholine, 1,2-di-O-hexylphosphatidylcholine and mixtures thereof.

Instead or in addition to short chain phospholipids the bicelles may comprise at least one detergent, which is preferably selected from the group consisting of 3-[(3-chloramidopropyl)dimethylammonio]-1-propane-sulfate (CHAPS), dicaproylphosphatidylcholine (DHPC), dicapryloylphosphatidylcholine (DCPC), dicaprylphosphatidylcholine, 1,2-di-O-hexylphosphatidylcholine and mixtures thereof.

The detergent used may not alter the properties of the protein to be incorporated in the lipid membrane and of course not inhibit the formation of bicelles.

An important aspect when using detergents in bicelles which are intended to be used in the production of lipid membranes is the ability of the detergent to non affect the native properties of an enzyme or protein, which will get in contact with said lipid membranes.

Another aspect of the present invention relates to a kit for the manufacturing of a supported lipid membrane comprising:
- bicelles and/or components involved in bicelle formation,
- S-layer proteins, and
- optionally a substrate.

The S-layer proteins of said kit are preferably provided in a stabilised form, preferably in a lyophilised form. The components involved in bicelle formation may comprise at least one short chain and at least one long chain phospholipid and/or at least one detergent as defined herein. Of course also tetraetherlipids may be employed.

Another aspect of the present invention relates to a lipid membrane or solid supported lipid membrane obtainable by a method according to the present invention.

It surprisingly turned out that a lipid membrane obtainable by a method according to the present invention shows enhanced features over similar membranes obtainable by conventional methods, like Langmuir-Blodgett or liposome fusion techniques.

Bicelles applied on a substrate covered with peptides, polypeptides, proteins or the like resulted in the formation of lipid membrane with a substantially continuous thickness and substantially without the presence of lipid aggregates or unspecific binding of excessive bicelles. Furthermore, the lipid membrane obtained covered at least 90%, preferably at least 95%, more preferably at least 99%, most preferably 100%, of the substrate surface comprising peptides, polypeptides, proteins or the like. The overall coverage of the substrate with lipid membranes can be observed, for instance, with AFM.

In contrast thereto, lipid membranes on substrates comprising peptides, polypeptides, proteins or the like obtainable with Langmuir-Blodgett techniques cover only approximately 75% of the surface of said substrates. Furthermore, such lipid membranes comprise areas with lipid multilayers and lipid aggregates (due to collapsed lipid bilayers, reorganisation and desorption processes) and do not show any insulating effect when applying electrochemical measurements (e.g. impedance spectroscopy, cyclic voltammetry) .

AFM imaging, electrochemical and surface plasmon resonance measurements revealed that the addition of liposomes on substrates covered with peptides, polypeptides, proteins or the like mainly resulted in adsorption rather than fusion processes. Fusion processes resulting in the formation of lipid bilayer structures could only be detected by electrochemical measurements on very small areas of the substrates (see also Naumann et al., 1999, 2002). The liposomes preferably adsorb, remain intact and form aggregates. Consequently the surface of the substrate is very irregular.

The lipids of the membrane according to the present invention may be modified with functional groups, which are preferably selected from the group consisting of biotinyl-, maleimide-, succinyl-, glutaryl-, carboxacyl-, and metal-chelating groups and combinations thereof, thus resulting in biotinyl-, maleimide-, succinyl-, glutaryl-, carboxacyl-, and metal-chelating lipids.

Yet another aspect of the present invention relates to the use of bicelles as defined herein for the manufacturing of a lipid membrane or a solid supported lipid membrane on a substrate covered at least in part with a protein layer.

It surprisingly turned out that especially bicelles can be suitably employed for the manufacturing of solid supported lipid membranes on a substrate which is at least in part covered with a protein layer.

The present invention is further illustrated by the following figures and examples, without being restricted thereto.

Fig. 1: Schematic drawing of a bicelle morphology. The central planar region is formed by long-chain phospholipids. The rim is stabilized by a short-chain phospholipids.

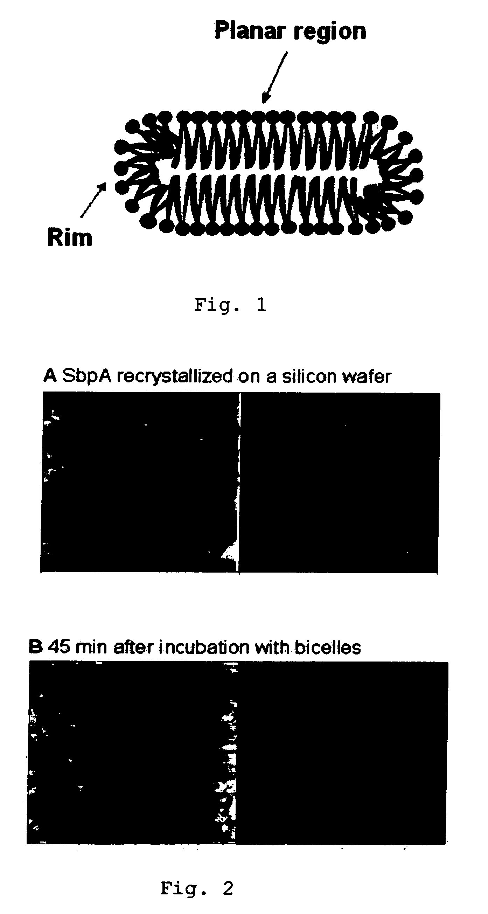

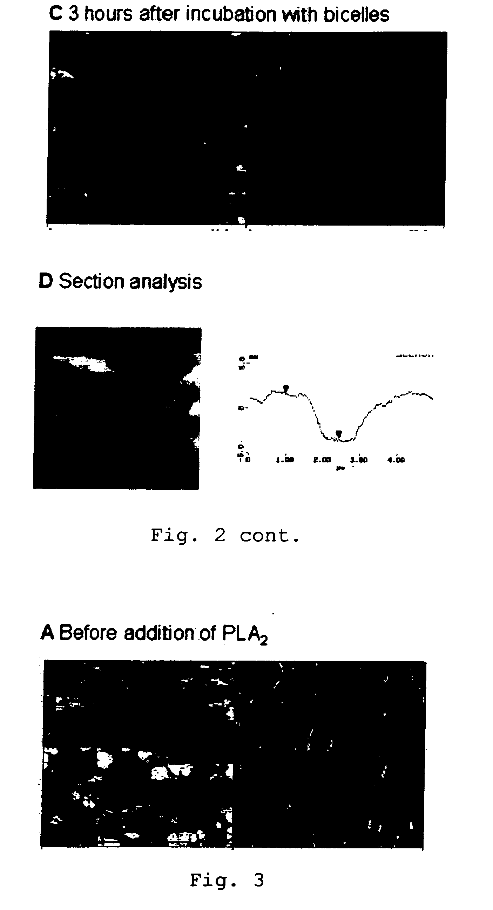

Fig. 2: Formation of an DMPC/DCPC-bilayer on an SbpA-covered silicon wafer by fusion of bicelles. (A) Recrystallized SbpA on hydrophilic silicon before addition of DMPC/DCPC-bicelles. The percentage of SbpA-coverage was 95-98%. The black spots were holes in the S-layer. (B) An SbpA/silicon chip after 45 min of incubation with DMPC/DCPC-bicelles showing membrane patches along the rim of the S-layer defects and crystallite boundaries. (C) SbpA/silicon chip after 3 hours of incubation with DMPC/DCPC-bicelles. Almost the entire chip was covered with a bilayer. (A,B,C) Left images: height mode (Z-range = 20 nm). Right images: deflection mode (Z-range = 8 nm). Scan size: 30 x 30 µm². (D) The section analysis revealed a bilayer thickness of ~ 5 nm. Scan size: 5 x 5 µm².

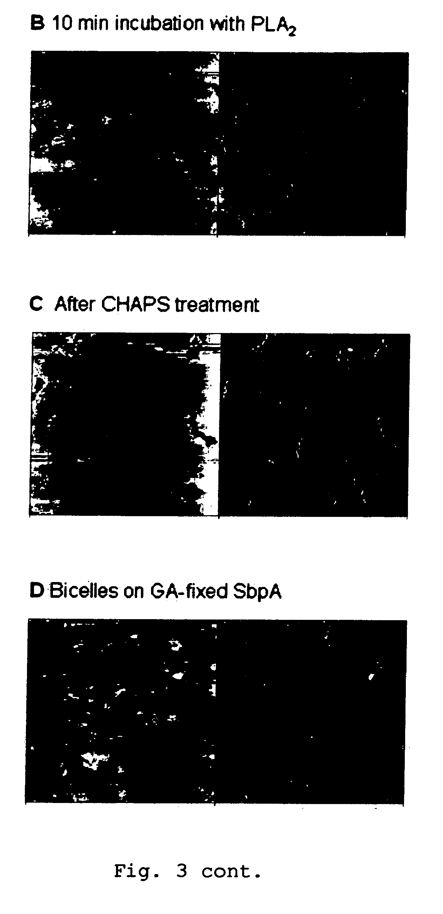

Fig. 3: SbpA/silicon supported EggPC-bilayers. Left images: height mode. Right images: Deflection mode. Scan size: 20 x 20 µm². Bilayer patches formed after 5 hours of incubation with EggPC-bicelles (A) before and (B) after incubation (10 min) with bee venom PLA₂. PLA₂ hydrolyzed the bilayer entirely. Z-range (height) = 20 nm; Z-range (deflection) = 10 nm. (C) EggPC-bilayer after CHAPS-treatment (first washing step): the bilayer could be removed entirely after several washing with 20 mM CHAPS solution. Z-range (height) = 15 nm; Z-range (deflection) = 8 nm. (D) EggPC-bilayer formed on glutaraldehyde-treated SpbA. The bicelles were incubated for 5 hours. Z-range (height) = 15 nm; Z-range (deflection) = 7 nm.

Fig. 4: Adsorption of EggPC-liposomes on SbpA/silicon chip after (A) 5 min and (B) 55 min incubation. Scan size = 30 x 30 µm². Z-range (height) = 20 nm; Z-range (deflection) = 12 nm.

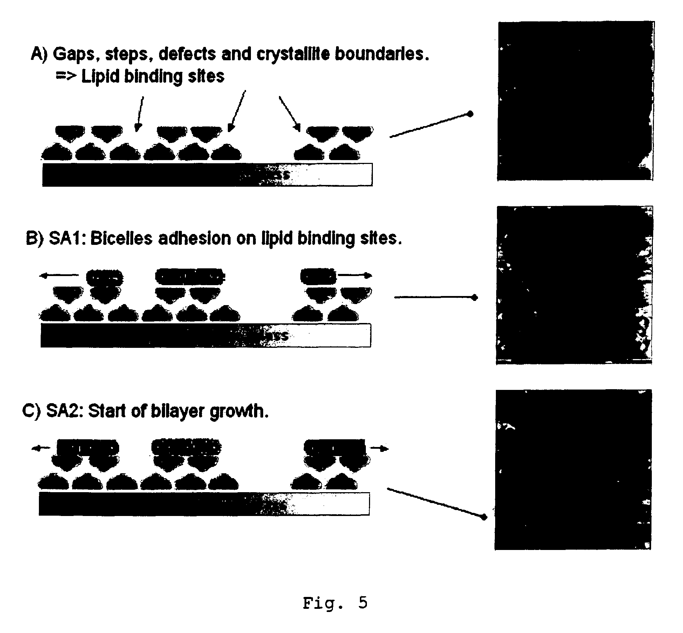

Fig. 5: Schematic illustrations of the bilayer formation process by bicelle fusion.(SA = self assembly).

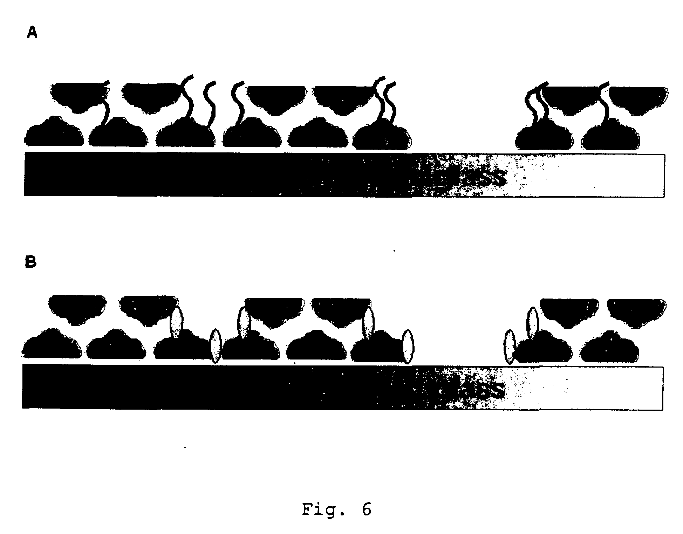

Fig. 6: (A) Schematic drawing of the SCWP-remnants on recrystallized SbpA. (B) Schematic drawing of the "repetitive hydrophobic pearl necklet" along the defect rims and crystallite boundaries.

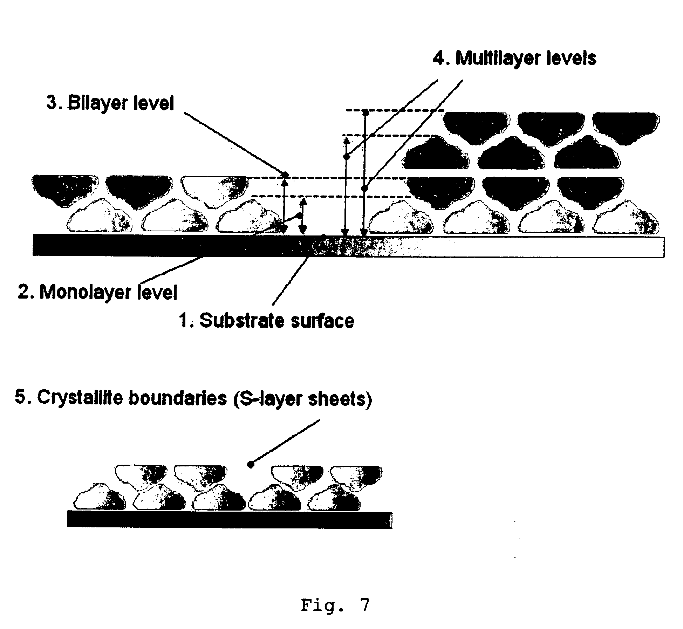

Fig. 7: Schematic drawing of gaps and steps within an recrystallized S-layer.

Fig. 8: shows a surface pressure-area isotherm (n-A isotherm) diagram. The surface pressure [mN/m] is plotted as a function of the area per molecule [nm²].

Fig. 9: shows schematically the formation of composite S-layer/lipid structures on solid supports by applying the Langmuir-Blodgett-technique. Technique (A) comprises the formation of a protein layer on a solid support followed by a vertical Langmuir-Blodgett-transfer of a lipid membrane formed at an air/water interface. Method (B) involves the formation of a lipid monolayer at an air/water interface, followed by the formation of a protein layer on said monolayer and transfer of the protein/lipid structure to a solid support.

Fig. 10: SbpA-supported DPPC-bilayer formed by Langmuir-Blodgett- and Langmuir-Schaeffer-techniques (LB-LS-technique).

Fig. 11: AFM-image of a composite S-layer/Tetraetherlipidstructure (SbpA/main tetraetherlipid (MPL) of the archeal organism Thermoplasma acidophilum) after transfer onto a silicon wafer with the LS-technique.

Fig. 12: Schematic drawing of the adsorption and partial fusion of liposomes on a solid support covered with a protein layer.

### Examples:

The following examples implement bicelle fusion on recrystallized S-layers as a seminal alternative to liposome fusion.

In these examples bicelles were prepared either with EggPC, which is a natural lipid mixture composed of long-chain and short-chain phosphatidylcholines or with a binary mixture of DM-PC (C14) and DCPC (C8). Silicon wafers covered with an ultrathin protein bilayer (and in some experiments protein monolayer) of the well-characterized S-layer protein SbpA from the gram-positive organism *Bacillus sphaericus* CCM 2177 were used as solid supports. SbpA (Mr = 129 kDa) forms a lattice with square lattice symmetry and a lattice constant of 13.1 nm (Ilk et al., 2002). One morphological unit (A ~ 170 nm²) is composed of four subunits. The pore size is - 3.5 nm. SbpA is not glycosylated. Typically for S-layer proteins, SbpA exhibits a corrugated net-positively charged inner surface and a smooth charge-neutral outer surface. SbpA recrystallizes on a great variety of surfaces such as silicon, gold, planar lipid films, liposomes and layers of secondary cell wall polymer (SCWP). Calcium ions are required for crystallization. SbpA recrystallizes in bilayers on hydrophilic silicon wafers and glass. On hydrophobic silicon wafers, gold and SCPW-layers SbpA forms monolayers (Györvary et al., 2003). In bilayers the inner, charged surfaces are attached to each other. In monolayers the proteins are oriented with their inner surface to the silicon. The thickness of an SbpA monolayer (~9 nm) was determined by scanning force microscopy (AFM) (Györvary et al., 2003) and X-ray reflectivity (Weygand et al., 1999). A thickness of ~15 nm was observed for SbpA bilayers recrystallized on solid supports (AFM) (Györvary et al., 2003). On solid supports the smoother outer surface is always exposed to the aqueous bulk solution. The rigid cell wall of gram-positive bacteria is composed of peptidoglycan and accessory SCWPs. It was shown that S-layer homologous (SLH) motifs, located at the N-terminal part of the polypeptide chain, anchor the proteins via SCWPs to the underlying cell wall (Ries et al., 1997). SbpA contains at least one SLH motif. SCWP from B. sphaericus CCM 2177 is a teichuronic acid, which is composed of disaccharide repeating units (Ilk et al., 1999).

### Materials:

The phospholipids egg phosphatidylcholine (EggPC; powder), 1,2-dioctanoyl-sn-glycero-3-phosphocholine (DCPC; C8:0; chloroformic solution) and 1,2-dimyristoyl-sn-glycero-3-phosphocholine (DMPC; C14:0; powder) were purchased from Avanti Polar Lipids (Alabaster, Alabama, USA). All chemicals were purchased from Sigma Aldrich (Vienna, Austria). Buffer solutions were prepared in MilliQ water (Millipore, minimum resistance > 18 Mohms cm). All buffer solutions were thoroughly degassed and filtered through 0.2 µm cellulose acetate filter (Sartorius AG, Göttingen, Germany) before use. Phospholipase A₂ from bee venom was purchased from Sigma (3.5 u/ml in 0.5 mM Tris (pH 8.5) containing 10 mM CaCl₂).

### Example 1: Isolation and recrystallization of S-layer proteins

Growth, cell wall preparations, and extractions of the S-layer protein SbpA isolated from B-sphaericus CCM 2177 (Czech Collection of Microorganisms) were performed as described in (Sleytr et al., 1986). The SbpA stock solution was diluted with 0.5 mM Tris (pH 9) containing 10 mM CaCl₂ to a final concentration of 0.1 mg/ml. Silicon wafers (7x7 mm) were purchased from NMRC (Cork, Ireland). The substrates were cleaned by rinsing them twice with absolute ethanol and MilliQ water. Subsequently, the substrates were dried in a stream of nitrogen. After treating the chips with UV/ozone in a plasma cleaner (PlasmaPrep2; Gala, Gabler Labor Instruments GmbH, Germany) the S-layer protein solution was added immediately for recrystallization. Recrystallization was performed for 4-6 hours. Atomic force microscopical (AFM) investigations (Nanoscope III, Digital Instruments Inc., Santa Barbara, CA) were performed to confirm the entire coverage of the gold surface with a crystalline SbpA lattice. In some experiments, the SbpA lattice was cross-linked with glutaraldehyde (20 min; 0.5% glutaraldehyde in 100 mM phosphate buffer, pH 7.5) or BS³ (1mg/ml in MilliQ water).

### Example 2: Preparation of bicelles and liposomes

*a) EggPC-bicelles:* 3mg/mL EggPC (powder) and 6mg/mL CHAPS were hydrated in a 10 mM Tris buffer (pH 7.4) containing 50 mM KCl. 100 mg Bio-Beads SM-2 (Sigma Aldrich, Vienna, Austria) were added per mL lipid/detergent solution. The solution was stirred and the OD₆₀₀ was measured photometrically. The solution was separated from the beads when the OD₆₀₀ reached ~ 0.9 - 1 (after ~ 35-40 min of stirring). The bicelles were filtered through a polycarbonate filter with a pore size of 200 nm before use. The bicelle solution was stored at 4°C and used within 2 weeks. The diameter of the EggPC bicelles was 40-50 nm (transmission electron microscopy of negatively stained samples).
*b) DMPC*/*DCPC-bicelles:* DCPC (chloroformic solution) was filled into a glass flask. The solvent was evaporated under a stream of nitrogen and stored in a vacuum chamber for at least 2 hours to remove the entire solvent. The lipid-cake was suspended in 10 mM Tris (pH 7.4) containing 50 mM KCl to a final concentration of 50 mg/ml. An appropriate amount of DMPC (powder) was added to the DCPC-suspension, such that the molar ratio of DMPC/DCPC was 3.5 (the molar ratio is known as the q-value). The concentration was 150 mg total lipid/ml. The DMPC/DCPC-mixture swelled for 24 hours. The sample was equilibrated by the following cycle: vigorous vortexing; cooling to 0°C (ice); heating to 40°C in a water bath. The cycle was repeated until a gel-like homogeneous solution was obtained. The solution was diluted to a final concentration of 15mg/ml, aliquoted and stored at -70°C until usage. The bicelle-solution was filtered through a polycarbonate filter with a pore size of 200 nm before use. The diameter of the DMPC/DCPC bicelles was 60 - 80 nm (transmission electron microscopy of negatively stained samples). For comparison, EggPC liposomes were prepared with the extrusion method followed by 5 freeze-and-thaw cycles. For extrusion a mini-extruder (Avanti Polar Lipids, Alabama, USA) and polycarbonate filters with a pore size of 100 nm were used (Nuclepore, Whatman, Kent, UK).

Silicon and S-layer-substrates were covered with one of the two bicelle-solutions and incubated at room temperature in a humidity chamber in order to prevent the sample from running dry. After the incubation time (30 min to 18 h) the samples were immersed in a 10 mM HEPES buffer (pH 7.4) containing 100 mM NaCl and imaged with AFM. The chips were regenerated by repeated washing in 20 mM CHAPS (dissolved) in MilliQ water and final rinsing with 10 mM HEPES, pH 7.4, 100 mM NaCl.

### Example 3: Atomic force microscopy (AFM)

AFM images were recorded in contact mode in liquid with a Nanoscope III Atomic Force Microscope (Digital Instruments, Santa Barbara, CA). Oxide-sharped silicon nitride tips (Nano-Probes, Digital Instruments) with a normal spring constant of 0.06 N/m were used. See results in Fig. 2 to 4.

### Example 4: Formation of lipid bilayers on SbpA-covered silicon wafers by fusion of bicelles

Both types of bicelles (EggPC- and DMPC/DCPC-bicelles) fused to lipid bilayers on SbpA. Lipid bilayer formation of DMPC/DCPC-bicelles was found to be slightly faster. Fig. 2A shows a silicon chip with a recrystallized SbpA-lattice. SbpA recrystallized on hydrophilic silicon as a bilayered S-layer lattice, forming large, very flat sheets. Very narrow gaps were detected along the boundaries of the upper S-layer sheets. The clearly visible dark spots in the height image were holes (0.5 - 1 µm in diameter) within the SbpA-double-layer. The surface occupancy ranged from 95 - 98 %. Fig. 2B and C showed AFM-images of a time sequence of a DMPC/DCPC-bilayer formation. After 45 min of incubation with DMPC/DCPC-bicelles (Fig. 2A) small bilayer patches along the rim of the S-layer defects and the crystallite boundaries could be detected. The defects in SpbA are considered to be the zones for initial bicelle adhesion (lipid binding sites). Initially bound bicelles acted as starting points for subsequent bilayer growth. After 3 hours of incubation with bicelles (Fig. 2B) most of the SbpA-surface was covered with a membrane. Only small holes in the bilayer were still visible. After 8-10 h of incubation the entire SbpA-lattice was covered with a membrane. The membrane did not span the S-layer holes. It was not possible to determine whether the membrane spanned the gap between two S-layer sheets or not with AFM. From height analyses a spanning bilayer over the gaps was assumed to be very likely. The thickness of the DMPC/DCPC membrane was ~ 5 nm (Fig. 2D). The samples could be stored for at least two weeks in buffer solution (10 mM HEPES buffer pH 7.4, 100 mM NaCl) at 4°C without membrane quality impairment.

### Example 5: Regeneration of the chip

The SbpA/silicon-supported lipid bilayer was easily displaced by phospholipase A₂-hydrolysis (Fig.3A and B) or repeated washing with 20 mM CHAPS detergent solution (Fig. 3C, image was taken after the first washing step). (Fig. 3A shows an AFM image of a supported EggPC-bilayer (after 5 hours of incubation). In this example the bilayer formation was not complete and the narrow gaps between the large bilayer sheets (= gaps along the boundaries of the S-layer sheets) are clearly visible. The thickness of EggPC-bilayers was ~ 5 nm. Fig. 3B shows the sample 10 min after incubation with bee venom PLA₂. PLA₂ catalyzes regio- and stereospecific hydrolysis of the sn-2 acyl ester linkage of sn-3-glyero-phospholipids (Verger, 1997) (Nielsen et al., 1999). 10 min after PLA₂ addition a strong bilayer degradation was observed. After 1 hour of incubation only a few lipid aggregates remained on the S-layer. The chips could be reused several times for lipid bilayer formation by adding a fresh bicelle solution.

### Example 6: Addition of bicelles to modified double-layered SbpA-lattices

In some experiments recrystallized SbpA was crosslinked with glutaraldehyde or the crosslinking agent BS³ prior to bicelles addition. After glutaraldehyde crosslinking a significant decrease in lipid bilayer formation especially along the S-layer defect-rims was observed (Fig. 3D). Crosslinking with BS³ did not have any impact on membrane formation. There was no visible difference to bilayers resting on unmodified SbpA. The only difference was that membrane formation was slightly slower requireing a longer incubation time (~12-15 hours).

### Example 7: Addition of bicelles to monolayered SbpA

Most of the examples in the present patent application deal with bilayered SbpA-lattices (recrystallized on hydrophilic silicon). For comparison bicelles were added to monolayered SbpA (recrystallized on hydrophobic silicon wafers, i.e. wafers without UV/ozone treatment prior to recrystallization). In contrast to a bilayered SbpA-lattice, a monolayered SbpA-lattice did not exhibit holes or defects (Györvary et al, 2003). The crystal sheets were very close together. Neither binding of bicelles nor lipid bilayer formation could be detected on monolayered SbpA.

### Example 8: Binding of modified biclles on S-layer monolayers

To facilitate the bicelle binding on the S-layer protein lattice, the system of SbpA-streptavidin-heterotetramer and biotinylated bicelles was investigated. Streptavidin is a tetrameric protein with four binding pockets for biotin. The biotin/streptavidin bond is one of the strongest non-covalent affinity interactions. Based on SbpA of *Bacillus sphaericus* CCM 2177, a recombinant S-layer-streptavidin fusion protein has been designed, expressed in *E. coli,* isolated and refolded to obtain functional heterotetramers (one chain S-layer fusion protein, plus three chains core streptavidin) (Moll et al., 2002). These SbpA-streptavidin fusion proteins have been recrystallized on substrates and subsequently incubated with biotinylated bicelles. Biotinylated bicelles have been prepared by mixing egg-PC with 2 mol% biotinylated lipid (1,2-dipalmitoyl-sn-glycero-3-phosphoethanolamine-N-(cap-biotinyl)) and CHAPS. As determined by AFM, lipidic patches could be demonstrated after one minute incubation indicating that biotinylated bicelles have been bound on the S-layer fusion protein.

### Example 9: Characterization of solid supported lipid membranes formed by three different membrane formation techniques

### (i) Bicelle fusion:

The membranes obtained by bicelle fusion were very flat (surface roughness 0.2-0.7 nm) exhibiting a continuous thickness of ~ 5 nm (depending on the used lipid species). The entire S-layer was covered with a single lipid bilayer (no lipid multilayers). Small gaps between large S-layer sheets were spanned by the membrane. Thus, the cover of the support with lipid bilayer related to the recrystallization quality of the S-layer (current surface coverage of double-layered SbpA on silicon chip: 90-98%), i.e. 100% cover of a solid substrate with an S-layer meant 100% cover of a single lipid membrane. The membranes were very clean (no adhered lipid aggregates or unspecific binding of excessiv bicelles).

### (ii) Langmuir-Blodgettry:

Langmuir-films are monomolecular assembled arrays of amphipathic molecules at the air/water interface (Ulman, 1991). A lipid solution (dissolved in a volatile solvent, e.g. chloroform) is spread upon the water surface (subphase) of a Langmuirteflon-trough. After evaporation of the solvent, a monolayer of molecules is left at the water surface.

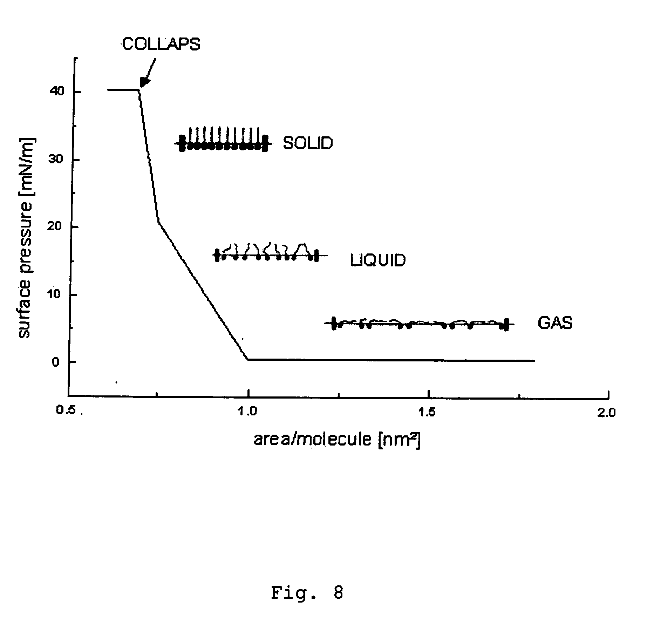

When the molecules are spread on the surface they are very loosely packed and form a two-dimensional "gas state". The area on the water available for each molecule is large and the surface pressure is low. The surface pressure can be increased by means of one or two sliding barriers (the surface area available to each molecule decreases). The first phase transition is from the "gas state" to the "liquid state" which is also known as liquid-expanded state (LE). With further compression the monolayer passes from the *liquid state* into the close packed *solid* state (liquid condensed state, LC). Further compression leads to the collaps of the monolayer due to mechanical instability. The surface pressure [mN/m], which is usually measured with a Wilhelmy plate sensor system, is plotted as a function of the area per molecule [nm²]. This plot is referred to as *surface pressure - area isotherm* (n-A isotherm; see Fig. 8).

The n-A isotherm provides important information in terms of monolayer stability, phase transitions, reorientation of molecules in the two-dimensional system and conformational transformations. The phase behaviour of the monolayer depends mainly on the physical and chemical properties of the amphiphilic molecules, and on the subphase temperature and composition. Many phospholipids have an additional transition phase (L2-L1) between the LE and the LC phase. The position (surface pressure value) of this transition phase strongly depends on the temperature. At higher temperatures the position appears at higher surface pressure values and vice versa (Roberts, 1990).

Once the molecules are compressed to the desired organization, the film can be transferred to a solid support by dipping the substrate through the monolayer (Blodgett, 1938). Mono- or multilayers transferred from the air/water-interface are referred to as *Langmuir-Blodgett films* (LB-films). Usually, the LB-transfer is carried out in the *solid state,* where the surface pressure is high enough to ensure a sufficient cohesion of the molecules in the monolayer. The monolayer is kept at a constant surface pressure by means of the barriers throughout the dipping process. Important parameters are the surface pressure during the deposition, the deposition speed and the type and nature of the solid substrate. The ratio between the decrease in the monolayer area on the air/water-interface during the deposition and the area of the substrate covered with the transferred monolayer is termed "transfer ratio". The transfer ratio is a dimension for the quantity of the deposited monolayer (ideal value is 1) (Roberts, 1990; Ulman, 1991).

The horizontal lifting of a monolayer from the air/water-interface is termed *Langmuir-Schaefer-transfer.* A compressed monolayer is prepared in a Langmuir-trough and the substrate is placed horizontally on the monolayer film. By lifting the substrate, the monolayer is transferred onto the substrate.

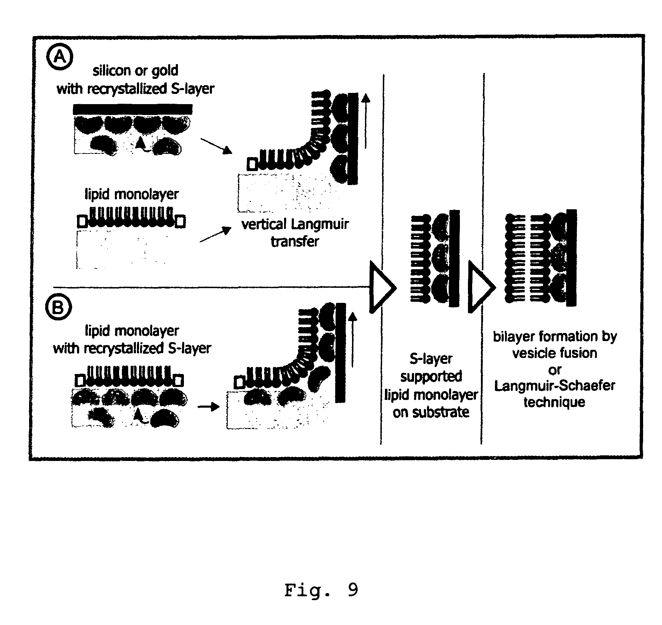

The formation of composite S-layer/lipid structures on solid supports can be performed by different strategies: by the Langmuir-Blodgett-technique in combination with the Langmuir-Schaefer technique or by vesicle (liposome) fusion, direct liposome fusion or by a modified Langmuir-Blodgett technique (Gufler et al., 2004) (see Fig. 9).

The first strategy (A) comprises the recrystallization of an S-layer lattice directly on a solid support (gold, silicon). The S-layer should cover the entire surface, which is carefully monitored by AFM. The first leaflet of the bilayer is formed at the air/water interface of a Langmuir-trough and subsequently deposited onto the S-layer covered substrate by a vertical Langmuir-Blodgett-transfer. The second leaflet is formed either by the Langmuir-Schaefer technique or by vesicle fusion. The latter method allows the simultaneous reconstituion of functional molecules by fusion of proteoliposomes. In the second approach (B) a lipid monolayer is formed at the air/water interface of a Langmuir-trough. The S-layer proteins are injected into the subphase and recrystallize on the lipid film. Subsequently, the composite S-layer/lipid structure is vertically transferred from the air/water-interface onto the substrate. The bilayer is completed by forming the second leaflet via Langmuir-Schaefer- or vesicle fusion- techniques.

Additionally to phospholipids, membrane-spanning tetraetherlipids are used. With the latter, only the first lipid deposition step (vertical Langmuir-Blodgett transfer) is necessary.

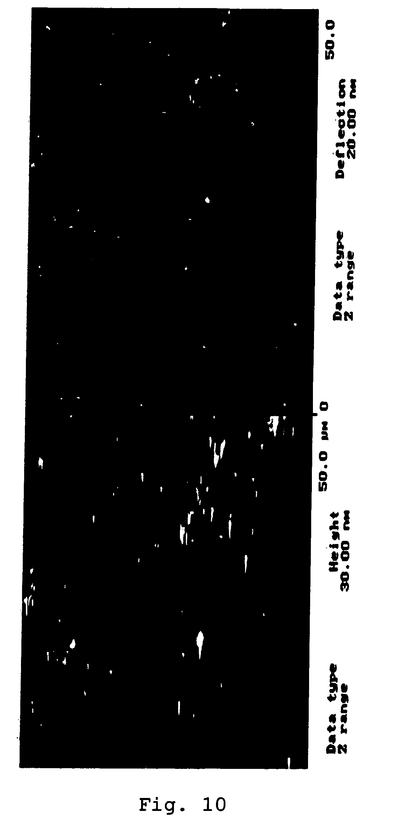

AFM-images of the transfer of lipid films onto S-layer covered supports (strategy A) showed round-shaped holes in the range from 0.3-2 µm in diameter within the membranes. A total membrane coverage of approximately 75% to 80% of the S-layer-covered substrate was observed. The membrane thickness was determined to be ~ 6 nm for a DPPC bilayer. Areas with lipid multilayers and lipid aggregates (due to collapsed lipid bilayers, reorganization and desorption processes) were also detected. The main problem was the insufficient transfer of closed films by the LB-LS-technique. The membrane fabrication process was time-consuming and not reproducible, therefore the quality of the product underlied strong variations. The addition of liposomes in order to fill up the holes in the membrane by the liposome fusion method did not lead to any improvement in membrane quality (only liposome adsorption). The insulating effect of the membranes was not detectable with electrochemical measurements (impedance spectroscopy, cyclic voltammetry) (Gufler, 2004; Wetzer, 1997) (see Fig. 10).

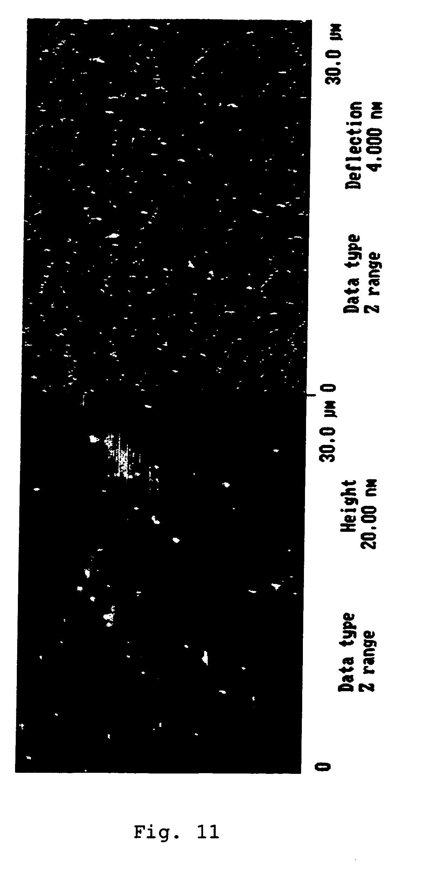

Experiments made with strategy B (transfer of S-layer/lipid structures onto a solid support) revealed that S-layer/lipid structures could not be transferred without destroying the structure. Therefore strategy B is not suited for the preparation of S-layer supported lipid membranes (see Fig. 11).

### (iii) Liposome fusion:

Liposomes are spherical lipid-bilayer bounded vesicles. They are formed by hydrating thin dried lipid films or lipid cakes in an aqueous solution. At first, the hydrated lipids form "onion-like" large, multilamellar vesicles (MLVs). Once these particles have formed, large unilamellar vesicles (LUVs) with reduced and uniform size can be produced by extrusion through a polycarbonate filter with a distinct diameter (50-200 nm and larger) or by dilution of a detergent. Preceding alternating freezing and thawing cycles of MLVs facilitate the extrusion. By ultrasonication of MLVs small unilamellar vesicles (SUVs) are obtained (diameter: 15-50 nm).

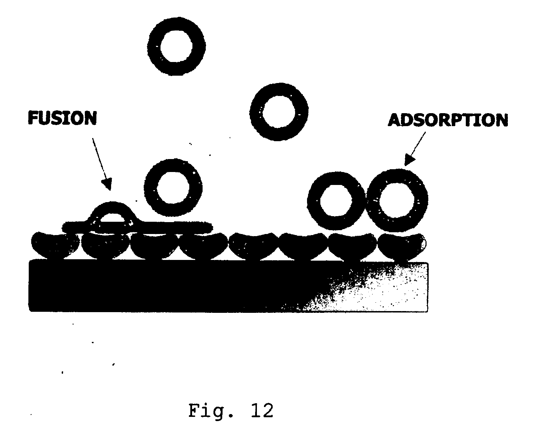

Formation of bilayers on hydrophilic supports by spreading and fusion of vesicles has been reported in many studies (Kalb et al., 1992; Leonenko et al., 2000; Nollert et al., 1995; Reviakine & Brisson, 2000; Jass et al., 2000). Liposomes can adsorb on a surface and either remain intact (adsorption => supported vesicle layer or vesicle aggregates), or they can rupture and form a planar supported bilayer (fusion) (Nollert et al., 1995; Leonenko et al., 2000). The adsorption, fusion and desorption kinetics strongly depend on the nature of the lipid or lipid mixture, the composition of the buffer and the solid supports (Kalb et al., 1992) (see Fig. 12).

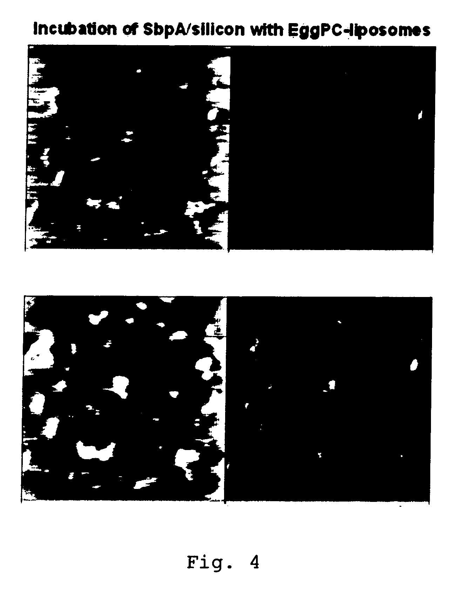

AFM imaging (see Fig. 4), electrochemical and surface plasmon resonance measurements revealed that the addition of liposomes mainly resulted in adsorption processes on S-layers. Different types of liposomes and S-layers were used. Fusion processes were detected with electrochemical measurements, which showed that only a very small area of the chip was covered with a bilayer, presumably membrane patches. The liposomes preferably adsorbed and remained intact and formed aggregates. Nevertheless, selective valinomycin-mediated transport of K⁺-ions across a membrane could be demonstrated (Gufler, 2004). It could be demonstrated by QCM-D that liposomes predominantly adsorbed on S-layers. Liposome fusion was also investigated with AFM. After incubation of the S-layer-covered substrates with the liposomes (over night), variable amounts of adsorbed liposomes and liposomes aggregates were detected. This was observed already previously with freeze-dried samples in TEM (Wetzer, 1997). Rarely, areas with bilayer patches could be detected (quantitative information not possible with AFM). The substrate surface was very irregular and covered with adsorbed liposome-aggregates.

Addition of liposomes to peptide supports (Naumann et al., 1999, 2002) showed insufficient electrochemical properties, similar to those obtained with S-layers. Adsorption processes prevailed. In spite of the poor quality of peptide-tethered bilayers, bovine cytochrome c oxidase and H⁺-ATP synthase from chloroplasts were incorporated in a functionally active form and investigated by impedance spectroscopy.

### References:

Blodgett, K.B. (1938) Journal of the American Chemical Society 57:1007-1022.
Cornell, B.A., et al. (1997). Nature 387, 580-583.
Faham, S. and Bowie, J.U. (2002). J Mol. Biol. 316, 1-6.
Faham, S., et al. (2005). Protein Sci. 14, 836-840.
Glover, K.J., et al. (2001). Biophys. J 81, 2163-2171.
Gufler, P., et al. (2004). Biochim. Biophys. Acta 1661, 154-165.
Gufler, P. (2004). S-layer supported functional lipid membranes. Dissertation. Univ. f. Bodenkultur, Wien.
Györvary, E.S., et al. (2003). J Microscopy 212, 300-306.
Ilk, N., et al. (2002). Appl. Environm. Microbiol. 68, 3251-3260.
Jass, J., et al. (2000). Biophys. J. 79, 3153-3163.
Kalb, E., et al. (1992). Biochim. Biophys. Acta 1103, 307-316.
Keller, C.A. and Kasemo, B. (1998). Biophys. J 75, 1397-1402.
Knoll, W., et al. (2004) In: Ultrathin Electrochemical Chemo- and Biosensors, Wolfbeis, O.S., Mirsky, V.M. (Eds.), pp. 239-253, Springer Verlag, Berlin, Germany.
Krishna, G., et al. (2003) Langmuir 19, 2294-2305.
Lang, H., et al. (1994) Langmuir 10, 197-210.
Leonenko, Z.V., et al. (2000). Biochim. Biophys. Acta 1509, 131-147.
Moll, D., et al. (2002) Proc Natl Acad Sci USA, 99, 14646-14651.
Naumann, R., et al. (2002). Biosens. Bioelectron. 17, 25-34.
Naumann, R., et al. (2003). Langmuir 19, 5435-5443.
Nielsen, L.K., et al. (1999). Biochim. Biophys. Acta 1420, 266-271.
Nollert, P., et al. (1995). Biophys. J. 69, 1447-1455.
Peggion, C., et al. (2001) Langmuir 17, 6585-6592.
Pum, D. and Sleytr, U.B. (1995). Colloids & Surfaces A: Physicochemical & Engineering Aspects 102, 99-104.
Raffard, G., et al. (2000). Langmuir 16, 7655-7662.
Ram, P. and Prestegard, J.H. (1988). Biochim. Biophys. Acta 940, 289-294.
Raguse, B., et al. (1998) Langmuir 14, 648-659.
Reimhult, E., et al. (2003). Langmuir 19, 1681-1691.
Reviakine, I. and Brisson, A. (2000). Langmuir 16, 1806-1815.
Ries, W., et al. (1997). Journal of Bacteriology 179, 3892-3898.
Roberts, G. (ed.). 1990. Langmuir-Blodgett Films. Plenum Press:New York.
Sackmann, E. (1996). Science 271, 43-48.
Sanders, C.R. and Prestegard, J.H. (1990). Biophys. J. 58, 447-460.
Sanders, C.R. and Landis, G.C. (1995). Biochemistry 34, 4030-4040.
Sasaki, H., et al. (2003). Bioorg. Med. Chem Lett. 13, 3583-3585.
Schiller, S.M., et al. (2003). Angew. Chem. Int. Ed Engl. 42, 208-211.
Schuster, B., et al. (1998). Biochim. Biophys. Acta 1370, 280-288.
Schuster, B., et al. (2001). Langmuir 17, 499-503.
Schuster, B., et al. (2003). Langmuir 19, 2392-2397.
Seifert, U. and Lipowsky, R. (1991). Molecular crystals and liquid crystals 202, 17-25.
Seitz, M., et al. (2000). Langmuir 16, 6067-6070.
Sleytr, U.B., et al. (1986). Arch. Microbiol. 146, 19-24.
Sleytr, U.B. and Sára, M. (1986). Applied Microbiology & Biotechnology 25, 83-90.
Struppe, J., et al. (2000). Biophys. J 78, 281-289.
Stryer, L. (1995). Biochemistry. 4th edition. W.H. Freeman and Company: New York.
Ulman,A. 1991. An introduction to ultrathin organic films. From Langmuir-Blodgett to self-assembly. Academic Press:San Diego.
Verger, R. (1997). Trends in Biotechnology 15, 32-38.
Wetzer, B. (1997). Rekristallisation und Charakterisierung von S-Schichten und ihre Anwendung zur Herstellung biomimetischer Membranen. Dissertation. Univ. f. Bodenkultur, Wien.
Weygand, M., et al. (1999). Biophys. J 76, 458-468.
Whiles, J.A., et al. (2002). Bioorg. Chem 30, 431-442.

## Claims

1. Method for the manufacturing of a supported lipid membrane comprising the steps:
- providing a substrate, covered at least in part with a layer comprising proteins, glycoproteins, polypeptides and/or peptides,
- contacting and incubating said substrate with a solution comprising bicelles, which comprise at least one short chain and at least one long chain phospholipid and/or at least one detergent, thereby producing a supported lipid membrane, and
- optionally removing the lipid membrane formed on the substrate from said substrate.

2. Method according to claim 1, **characterised in that** the protein, glycoprotein polypeptide or peptide layer is modified with at least one functional group.

3. Method according to claim 2, **characterised in that** the at least one functional group is selected from the group consisting of proteins, polypeptides, peptides, glycans and mixtures thereof.

4. Method according to any one of claims 1 to 3, **characterised in that** the protein and/or glycoprotein layer is an S-layer.

5. Method according to any one of claims 1 to 4, **characterised in that** the substrate is substantially planar.

6. Method according to any one of claims 1 to 5, **characterised in that** the substrate comprises a material selected from the group consisting of silicon, semiconducting material, in particular semiconducting silicon, gallium arsenide and aluminum gallium arsenide, organic or inorganic polymers, solid state polymers and mixtures thereof.

7. Method according to any one of claims 1 to 6, **characterised in that** the surface of the substrate is coated with a metal and/or metal oxide.

8. Method according to claim 7, **characterised in that** the metal is gold or aluminium and the metal oxide is indium tin oxide (ITO).

9. Method according to any one of claims 1 to 8, **characterised in that** the surface of the substrate is chemically or physically modified.

10. Method according to claim 9, **characterised in that** the modification is selected from the group consisting of ionizing radiation, atomic radicals, corona treatment, silane groups, functional groups and mixtures thereof.

11. Method according to claim 10, **characterised in that** the functional group is a glycan chain, in particular a secondary cell wall polymer.

12. Method according to any one of claims 1 to 11, **characterised in that** the at least one long chain phospholipid comprises fatty acid residues with 10 to 24, preferably 12 to 20, more preferably 14 to 18, carbon atoms.

13. Method according to any one of claims 1 to 12, **characterised in that** the at least one long chain phospholipid is selected from the group consisting of dimyristoylphosphatidylcholine (DM-PC), dimyristelaidoylphosphatidylcholine, myristoylpalmitoylphosphatidylcholine, dipalmitoylphosphatidylcholine (DPPC), 1,2-bis(10,12-tricosadiynoyl)-sn-glycero-3-phosphocholine, di-O-tetradecylphosphatidylcholine, egg-PC, di-O-hexadecylphosphatidylcholine and mixtures thereof.

14. Method according to claim 31, **characterised in that** the at least one long chain phospholipid is at least in part substituted with at least one tetraether lipid selected from the group consisting of caditocalarchaeol tetraetherlipids, in particular glycerol-dialkyl-nonitol-tetreather lipid (GDNT) and glycerol-dialkyl-glycerol-tetreather lipid (GDGT), calarchaeol tetraetherlipids and mixtures thereof.

15. Method according to any one of claims 1 to 14, **characterised in that** the at least one short chain phospholipid comprises fatty acid residues with 4 to 10, preferably 6 to 8, carbon atoms.

16. Method according to any one of claims 1 to 15, **characterised in that** the at least one short chain phospholipid is selected from the group consisting of dicaproylphosphatidylcholine (DHPC), dicapryloylphosphatidylcholine (DCPC), dicaprylphosphatidylcholine, 1,2-di-O-hexylphosphatidylcholine and mixtures thereof and mixtures thereof.

17. Method according to any one of claims 1 to 16, **characterised in that** the at least one detergent is selected from the group consisting of 3-[(3-chloramidopropyl)dimethylammonio]-1-propanesulfate (CHAPS), 3-[(3-cholamidopropyl)dimethylammonio]-2-hydroxy-1-propanesulfonate (CHAPSO), dodecyldimethyl-N-amineoxide (DDAO), cetyltrimethylammonium bromide (CTAB), Bis(2-ethylhexyl)sulfosuccinate sodium salt, N-lauroylsarcosine sodium salt, n-octyl-β-D-glucopyranoside (OG), n-dodecyl-β-G-maltoside (DDM), sodium cholate, sodium deoxycholate (DOC) and mixtures thereof.

18. Kit for the manufacturing of a supported lipid membrane comprising:
• bicelles and/or at least one long chain phospholipid and/or at least one short chain phospholipid and/or at least one detergent as defined in any one of claims 12 to 17,
• S-layer proteins, and
• optionally a substrate as defined in any one of claims 5 to 9.

19. Kit according to claim 18, **characterised in that** the S-layer proteins are provided in a stabilised form, preferably in a lyophilised form.

20. Lipid membrane or solid supported lipid membrane obtainable by a method according to any one of claims 1 to 17.

21. Membrane according to claim 20, **characterised in that** the lipids are modified with at least one functional group.

22. Membrane according to claim 21, **characterised in that** the at least functional group is selected from the group consisting of biotinyl-, maleimide-, succinyl-, glutaryl-, carboxacyl-, and metal-chelating groups and combinations thereof.

23. Use of bicelles as defined in any one of claims 1 to 16 for the manufacturing of a lipid membrane or a solid supported lipid membrane on a substrate covered at least in part with a protein layer.
